# EUROPÄISCHE PATENTSCHRIFT

(11) **EP 1 114 038 B1**
(45) Veröffentlichungstag und Bekanntmachung des Hinweises auf die Patenterteilung: **03.12.2003**
(21) Anmeldenummer: 99969089.4
(22) Anmeldetag: 09.09.1999
(51) Int. Cl.: C07D 275/03, C07D 417/12, A01N 43/80

(54) **ISOTHIAZOLCARBONSÄUREAMIDE**
ISOTHIAZOLE CARBOXYLIC ACID AMIDES
AMIDES D'ACIDE CARBOXYLIQUE D'ISOTHIAZOL

(30) Priorität: 16.09.1998 DE 19842354
(43) Veröffentlichungstag der Anmeldung: 11.07.2001
(73) Patentinhaber: Bayer CropScience AG, 40789 Monheim (DE)
(72) Erfinder: ASSMANN, Lutz, D-25826 St. Peter Ording (DE); ELBE, Hans-Ludwig, D-42329 Wuppertal (DE); KUHNT, Dietmar, D-51399 Burscheid (DE); HÄNSSLER, Gerd, D-51381 Leverkusen (DE); KUCK, Karl-Heinz, D-40764 Langenfeld (DE); KITAGAWA, Yoshinori, Moka-shi, Tochigi 321-4305 (JP); SAWADA, Haruko, Yuki-shi, Ibaraki 307-0007 (JP); SAKUMA, Haruhiko, Oyama-shi, Tochigi 323-0829 (JP)
(86) Internationale Anmeldenummer: EP9906649
(87) Internationale Veröffentlichungsnummer: WO00015622

(56) Entgegenhaltungen:
- EP-A- 0 313 091
- WO-A-99/24413
- DE-A- 1 770 976
- US-A- 5 240 951
- CHEMICAL ABSTRACTS, vol. 119, no. 11, 13. September 1993 (1993-09-13) Columbus, Ohio, US; abstract no. 117242x, Seite 933; XP002126109 & JP 05 059024 A (MITSUI TOATSU CHEMICALS) 9. März 1993 (1993-03-09)
- CHEMICAL ABSTRACTS, vol. 120, no. 21, 23. Mai 1994 (1994-05-23) Columbus, Ohio, US; Seite 398; XP002126110 & JP 06 009313 A (MITSUI TOATSU CHEMICALS) 18. Januar 1994 (1994-01-18) in der Anmeldung erwähnt

## Beschreibung

Die vorliegende Erfindung betrifft neue Isothiazolcarbonsäureamide, mehrere Verfahren zu deren Herstellung und deren Verwendung zum Schutz von Pflanzen gegen Befall durch unerwünschte Mikroorganismen und tierische Schädlinge.

Es ist bereits bekannt geworden, daß zahlreiche Isothiazolcarbonsäure-Derivate fungizide Eigenschaften besitzen (vgl. US-A 5 240 951, EP-A 0 313 091, JP-A 05-059 024, WO 99-24 413 und JP-A 06-009 313). So lassen sich z.B. 3,4-Dichlor-isothiazol-5-carbonsäure-furfurylamid und 3,4-Dichlor-isothiazol-5-carbonsäure-morpholinid zur Bekämpfung von Pilzen einsetzen. Die Wirksamkeit dieser Stoffe ist gut, läßt aber bei niedrigen Aufwandmengen in manchen Fällen zu wünschen übrig.

Es wurden nun neue Tsothiazolcarbonsäureamide der Formel 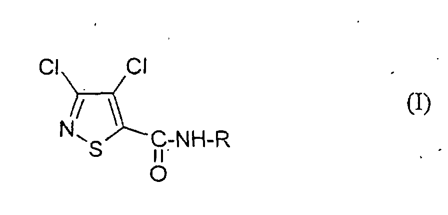 in welcher
- R: für einen Rest der Formel

-A¹-R⁵

steht, worin
A¹ für -CH₂-, -CH₂-CH₂- oder steht und 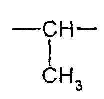
R⁵ für Thiazolidinthion-3-yl, Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl, 1,2,3-Triazolyl, Benzotriazolyl, Chinolinyl, Isochinolinyl, Chinazolinyl, Indolyl, Benzothienyl, Benzofuranyl, Benzothiazolyl oder Benzimidazolyl steht, wobei diese Reste einfach oder zweifach, gleichartig oder verschieden substituiert sein können durch Fluor, Chlor, Brom, Cyano, Nitro, Carboxyl, Methyl, Ethyl, Propyl, Isopropyl, n-Butyl, sek.-Butyl, iso-Butyl, tert.-Butyl, Halogenalkyl mit 1 oder 2 Kohlenstoffatomen und 1 bis 3 Fluor-, Chlor- und/oder Bromatomen, Methoxycarbonyl, Ethoxycarbonyl, Methoxy, Ethoxy, Propoxy, Isopropoxy, tert.-Butoxy, Halogenalkoxy mit 1 oder 2 Kohlenstoffatomen und 1 bis 3 Fluor-, Chlor- und/oder Bromatomen, Methylthio, Ethylthio, Halogenalkylthio mit 1 oder 2 Kohlenstoffatomen und 1 bis 3 Fluor-, Chlor- und/oder Bromatomen, Methylamino, Ethylamino, Dimethylamino, Diethylamino, Phenyl und/oder Phenoxy, oder
- R: für einen Rest der Formel 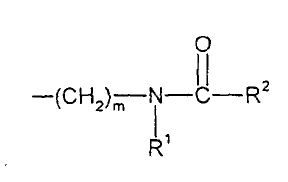 steht, worin
R¹ für Wasserstoff oder Alkyl mit 1 bis 4 Kohlenstoffatomen steht,
R² für Alkoxy mit 1 bis 4 Kohlenstoffatomen steht oder für Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl oder 1,2,3-Triazolyl steht, wobei diese Reste einfach oder zweifach, gleichartig oder verschieden substituiert sein können durch Fluor, Chlor, Brom, Cyano, Nitro, Carboxyl, Methyl, Ethyl, Propyl, Isopropyl, n-Butyl, sek.-Butyl, iso-Butyl, tert.-Butyl, Halogenalkyl mit 1 oder 2 Kohlenstoffatomen und 1 bis 3 Fluor-, Chlor- und/oder Bromatomen, Methoxycarbonyl, Ethoxycarbonyl, Methoxy, Ethoxy, Propoxy, Isopropoxy, tert.-Butoxy, Halogenalkoxy mit 1 oder 2 Kohlenstoffatomen und 1 bis 3 Fluor-, Chlor- und/oder Bromatomen, Methylthio, Ethylthio, Halogenalkylthio mit 1 oder 2 Kohlenstoffatomen und 1 bis 3 Fluor-, Chlor- und/oder Bromatomen, Methylamino, Ethylamino, Dimethylamino, Diethylamino, Phenyl und/oder Phenoxy,
und
m für 1 oder 2 steht, oder
- R: für einen Rest der Formel 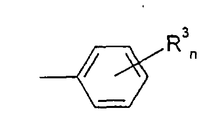 steht, worin
R³ für einen Heterocyclus der Formel 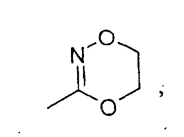 für Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl oder 1,2,3-Triazolyl swteht, wobei diese Reste einfach oder zweifach, gleichartig oder verschieden substituiert sein können durch Fluor, Chlor, Brom, Cyano, Nitro, Carboxyl, Methyl, Ethyl, Propyl, Isopropyl, n-Butyl, sek.-Butyl, iso-Butyl, tert.-Butyl, Halogenalkyl mit 1 oder 2 Kohlenstoffatomen und 1 bis 3 Fluor-, Chlor- und/oder Bromatomen, Methoxycarbonyl, Ethoxycarbonyl, Methoxy, Ethoxy, Propoxy, Isopropoxy, tert.-Butoxy, Halogenalkoxy mit 1 oder 2 Kohlenstoffatomen und 1 bis 3 Fluor-, Chlorund/oder Bromatomen, Methylthio, Ethylthio, Halogenalkylthio mit 1 oder 2 Kohlenstoffatomen und 1 bis 3 Fluor-, Chlor- und/oder Bromatomen, Methylamino, Ethylamino, Dimethylamino, Diethylamino, Phenyl und/oder Phenoxy
oder
R³ für einen Rest der Formel 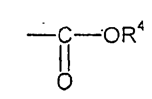 steht, worin
R⁴ für Cyclopropyl, Cyclopentyl oder Cyclohexyl steht
und
n für 1 oder 2 steht, oder
- R: für Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl, 1,2,3-Triazolyl, Benzotriazolyl, Chinolinyl, Isochinolinyl, Chinazolinyl, Indolyl, Benzothienyl, Benzofuranyl, Benzothiazolyl oder Benzimidazolyl steht, wobei diese Reste einfach oder zweifach, gleichartig oder verschieden substituiert sein können durch Fluor, Chlor, Brom, Cyano, Nitro, Carboxyl, Methyl, Ethyl, Trifluormethyl, Difluormethyl, Trichlormethyl, Methoxycarbonyl, Ethoxycarbonyl, Methoxy, Ethoxy, Trifluormethoxy, Methylthio, Trifluormethylthio, Methylamino, Dimethylamino, Phenyl und/oder Phenoxy,
gefunden.

Weiterhin wurde gefunden, daß sich Isothiazolcarbonsäureamide der Formel (I) herstellen lassen, indem man
a) 3,4-Dichlor-isothiazol-5-carbonsäurechlorid der Formel 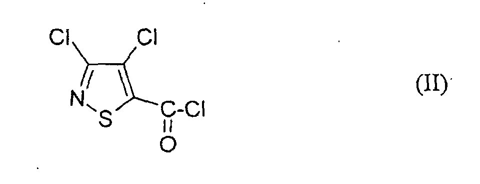 mit Aminen der Formel

   H₂N-R (III)

   in welcher
   - R: die oben angegebenen Bedeutungen hat,
   gegebenenfalls in Gegenwart eines Säurebindemittels und gegebenenfalls in Gegenwart eines Verdünnungsmittels umsetzt,
   oder
b) 3,4-Dichlor-isothiazol-5-carbonsäureamid der Formel 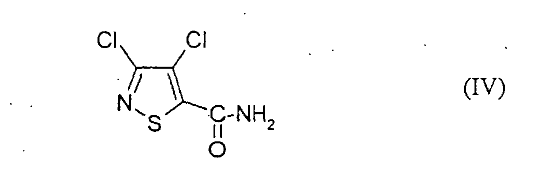 mit Hydroxyverbindungen der Formel

   HO-A-X (V)

   in welcher
   - X: für Thiazolidinthion-3-yl, Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl, 1,2,3-Triazolyl, Benzotriazolyl, Chinolinyl, Isochinolinyl, Chinazolinyl, Indolyl, Benzothienyl, Benzofuranyl, Benzothiazolyl oder Benzimidazolyl steht, wobei diese Reste einfach oder zweifach, gleichartig oder verschieden substituiert sein können durch Fluor, Chlor, Brom, Cyano, Nitro, Carboxyl, Methyl, Ethyl, Propyl, Isopropyl, n-Butyl, sek.-Butyl, iso-Butyl, tert.-Butyl, Halogenalkyl Halogenalkyl mit 1 bis 2 Kohlenstoffatomen und 1 bis 3 Fluor-, Chlor- und/oder Bromatomen, Methoxycarbonyl, Ethoxycarbonyl, Methoxy, Ethoxy, Propoxy, Isopropoxy, tert.-Butoxy, Halogenalkoxy mit 1 bis 2 Kohlenstoffatomen und 1 bis 3 Fluor-, Chlor- und/oder Bromatomen, Methylthio, Ethylthio, Halogenalkylthio mit 1 bis 2 Kohlenstoffatomen und 1 bis 3 Fluor-, Chlor- und/oder Bromatomen, Methylamino, Ethylamino, Dimethylamino, Diethylamino, Phenyl und/oder Phenoxy,
   oder
   - X: für einen Rest der Formel 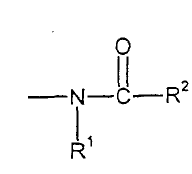 steht, worin
   R¹ und R² diejenigen Bedeutungen haben, die im Zusammenhang mit der Beschreibung der erfindungsgemäßen Stoffe der Formel (I) für diese Reste genannt wurden,
   und
   - A: für CH₂-, -CH₂-CH₂- oder 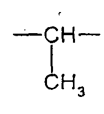 steht,
   in Gegenwart eines Verdünnungsmittels und in Gegenwart eines wasser-abspaltenden Mittels umsetzt.

Schließlich wurde gefunden, daß die Isothiazolcarbonsäureamide der Formel (I) sehr gut zum Schutz von Pflanzen gegen Befall durch unerwünschte Mikroorganismen verwendbar sind. Die erfindungsgemäßen Stoffe eignen sich sowohl zur Mobilisierung pflanzeneigener Abwehrkräfte gegen Befall durch unerwünschte Mikroorganismen als auch als Mikrobizide zur direkten Bekämpfung der Mikroorganismen.

Überraschenderweise besitzen die erfindungsgemäßen Stoffe eine bessere mikrobizide Wirksamkeit als 3,4-Dichlor-isothiazol-5-carbonsäure-furfurylamid und 3,4-Dichlor-isothiazol-5-carbonsäure-morpholinid, welches konstitutionell ähnliche, vorbekannte Wirkstoffe gleicher Wirkungsrichtung sind.

Die erfindungsgemäßen Isothiazolcarbonsäureamide sind durch die Formel (I) allgemein definiert.
- R: steht bevorzugt für einen Rest der Formel

-A¹-R⁵ ,

worin
A¹ für -CH₂-, -CH₂-CH₂- oder 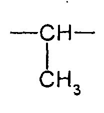 steht und
R⁵ für Thiazolidinthion-3-yl, Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl, 1,2,3-Triazolyl, Benzotriazolyl, Chinolinyl, Isochinolinyl, Chinazolinyl, Indolyl, Benzothienyl, Benzofuranyl, Benzothiazolyl oder Benzimidazolyl steht, wobei diese Reste einfach oder zweifach, gleichartig oder verschieden substituiert sein können durch Fluor, Chlor, Brom, Cyano, Nitro, Carboxyl, Methyl, Ethyl, Trifluormethyl, Difluormethyl, Trichlormethyl, Methoxycarbonyl, Ethoxycarbonyl, Methoxy, Ethoxy, Trifluormethoxy, Methylthio, Trifluormethylthio, Methylamino, Dimethylamino, Phenyl und/oder Phenoxy.
- R: steht außerdem bevorzugt für einen Rest der Formel 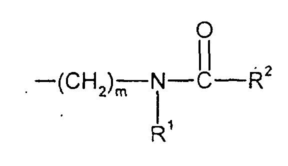 , worin
R¹ für Wasserstoff, Methyl oder Ethyl steht,
R² für Methoxy, Ethoxy oder Isopropoxy steht oder
R² für Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl, 1,2,3-Triazolyl steht, wobei diese Reste einfach oder zweifach, gleichartig oder verschieden substituiert sein können durch Fluor, Chlor, Brom, Cyano, Nitro, Carboxyl, Methyl, Ethyl, Trifluormethyl, Difluormethyl, Trichlormethyl, Methoxycarbonyl, Ethoxycarbonyl, Methoxy, Ethoxy, Trifluormethoxy, Methylthio, Trifluormethylthio, Methylamino, Dimethylamino, Phenyl und/oder Phenoxy,
und
m für 1 oder 2 steht.
- R: steht außerdem bevorzugt für einen Rest der Formel 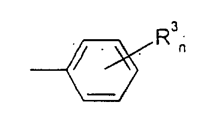 , worin
R³ für einen Heterocyclus der Formel 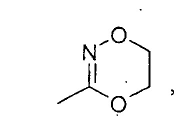 für Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl oder 1,2,3-Triazolyl steht, wobei diese Reste einfach oder zweifach, gleichartig oder verschieden substituiert sein können durch Fluor, Chlor, Brom, Cyano, Nitro, Carboxyl, Methyl, Ethyl, Trifluormethyl, Difluormethyl, Trichlormethyl, Methoxycarbonyl, Ethoxycarbonyl, Methoxy, Ethoxy, Trifluormethoxy, Methylthio, Trifluormethylthio, Methylamino, Dimethylamino, Phenyl und/oder Phenoxy,
oder
R³ für einen Rest der Formel 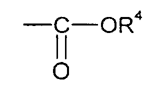 steht, worin
R⁴ für Cyclopentyl oder Cyclohexyl steht
und
n für 1 oder 2 steht.
- R: steht außerdem bevorzugt für für Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl oder 1,2,3-Triazolyl, Benzotriazolyl, Chinolinyl, Isochinolinyl, Chinazolinyl, Indolyl, Benzothienyl, Benzofuranyl, Benzothiazolyl oder Benzimidazolyl, wobei diese Reste einfach oder zweifach, gleichartig oder verschieden substituiert sein können durch Fluor, Chlor, Brom, Cyano, Nitro, Methyl, Trifluormethyl, Methoxycarbonyl, Methoxy, Trifluormethoxy, Methylthio und/oder Trifluormethylthio.

Die genannten Substituenten-Definitionen sind untereinander beliebig kombinierbar. Außerdem können auch einzelne Substituenten-Definitionen entfallen.

Verwendet man 3,4-Dichlor-isothiazol-5-carbonsäurechlorid und 4-Amino-morpholin als Ausgangsstoffe, so kann der Verlauf des erfindungsgemäßen Verfahrens (a) durch das folgende Formelschema veranschaulicht werden.

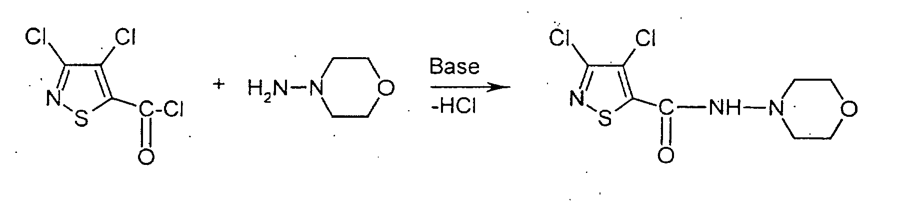

Verwendet man 3,4-Dichlor-isothiazol-5-carbonsäureamid und 3-Hydroxymethylthiazolidin-2-thion als Ausgangsstoffe, so kann der Verlauf des erfindungsgemäßen Verfahrens (b) durch das folgende Formelschema veranschaulicht werden.

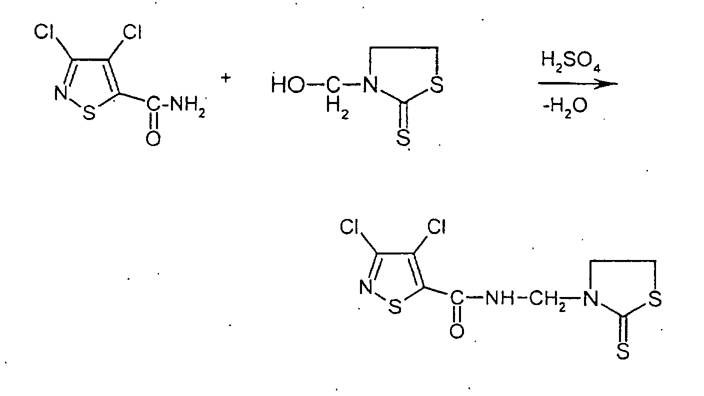

Das bei der Durchführung des erfindungsgemäßen Verfahrens (a) als Ausgangssubstanz benötigte 3,4-Dichlor-isothiazol-5-carbonsäure-chlorid der Formel (II) ist bekannt (vgl. US-A-5 240 951).

Die weiterhin bei der Durchführung des erfindungsgemäßen Verfahrens (a) als Reaktionskomponenten benötigten Amine sind durch die Formel (III) allgemein definiert. In dieser Formel hat R vorzugsweise diejenigen Bedeutungen, die bereits im Zusammenhang mit der Beschreibung der erfindungsgemäßen Stoffe der Formel (I) als bevorzugt für diesen Rest genannt wurden.

Die Amine der Formel (III) sind bekannt oder lassen sich nach bekannten Methoden herstellen.

Als Säurebindemittel kommen bei der Durchführung des erfindungsgemäßen Verfahrens (a) alle üblichen anorganischen und organischen Basen in Frage. Vorzugsweise verwendbar sind Erdalkalimetall- oder Alkalimetallhydride, -hydroxide, -amide, - alkoholate, -acetate, -carbonate oder -hydrogencarbonate, wie Natriumhydrid, Natriumamid, Natriummethylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat, ferner Ammoniumhydroxid, Ammoniumacetat oder Ammoniumcarbonat, oder tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (a) kommen alle inerten organischen Lösungsmittel in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen -10°C und +150°C, vorzugsweise zwischen 0°C und 100°C.

Bei der Durchführung des erfindungsgemäßen Verfahrens (a) arbeitet man im allge-. meinen unter Atmosphärendruck. Es ist aber auch möglich, unter erhöhtem oder, sofern keine flüchtigen Komponenten an der Umsetzung beteiligt sind, unter vermindertem Druck zu arbeiten.

Bei der Durchführung des erfindungsgemäßen Verfahrens (a) setzt man auf 1 Mol an 3,4-Dichlor-isothiazol-5-carbonsäure-chlorid der Formel (II) im allgemeinen 1 bis 5 Mol, vorzugsweise 1 bis 2 Mol an Amin der Formel (III) sowie eine äquivalente Menge oder einen Überschuß an Säurebindemittel ein. Die Aufarbeitung erfolgt nach üblichen Methoden. Im allgemeinen verfährt man in der Weise, daß man das Reaktionsgemisch nach beendeter Umsetzung einengt, den verbleibenden Rückstand mit Wasser und einem mit Wasser wenig mischbaren organischen Lösungsmittel versetzt, die organische Phase abtrennt, wäscht, trocknet und einengt. Das verbleibende Produkt kann nach üblichen Methoden von eventuell enthaltenen Verunreinigungen befreit werden.

Das bei der Durchführung des erfindungsgemäßen Verfahrens (b) als Ausgangssubstanz benötigte 3,4-Dichlor-isothiazol-5-carbonsäureamid der Formel (IV) ist bekannt (vgl. US-A-5 240 951 ).

Die weiterhin bei der Durchführung des erfindungsgemäßen Verfahrens (b) als Reaktionskomponenten benötigten Hydroxyverbindungen sind durch die Formel (V) allgemein definiert.
- X: steht bevorzugt für für Thiazolidinthion-3-yl, Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl, 1,2,3-Triazolyl, Benzotriazolyl, Chinolinyl, Isochinolinyl, Chinazolinyl, Indolyl, Benzothienyl, Benzofuranyl, Benzothiazolyl oder Benzimidazolyl steht, wobei diese Reste einfach oder zweifach, gleichartig oder verschieden substituiert sein können durch Fluor, Chlor, Brom, Cyano, Nitro, Carboxyl, Methyl, Ethyl, Trifluormethyl, Difluormethyl, Trichlormethyl, Methoxycarbonyl, Ethoxycarbonyl, Methoxy, Ethoxy, Trifluormethoxy, Methylthio, Trifluormethylthio, Methylamino, Dimethylamino, Phenyl und/oder Phenoxy.
- X: steht außerdem bevorzugt für einen Rest der Formel 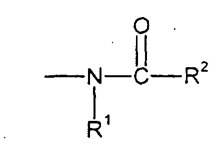 , worin
R¹ und R² diejenigen Bedeutungen haben, die im Zusammenhang mit der Beschreibung der erfindungsgemäßen Stoffe der Formel (I) für diese Reste als ganz besonders bevorzugt genannt wurden.
- A: steht bevorzugt für CH₂-, -CH₂-CH₂- oder

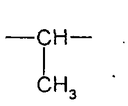

Die Hydroxyverbindungen der Formel (V) sind bekannt oder lassen sich nach bekannten Methoden herstellen.

Als Verdünnungsmittel kommen bei der Durchführung des erfindungsgemäßen Verfahrens (b) alle für derartige Umsetzungen üblichen, inerten organischen Solventien in Frage. Vorzugsweise verwendbar ist Eisessig.

Als wasserabspaltende Mittel kommen bei der Durchführung des erfindungsgemäßen Verfahrens (b) alle üblichen Reagenzien in Betracht, die zur Dehydratation befähigt sind. Vorzugsweise verwendbar sind Säuren, wie Schwefelsäure oder p-Toluolsulfonsäure, und auch Trockenmittel, wie wasserfreies Kieselgel.

Die Reaktionstemperaturen können auch bei der Durchführung des erfindungsgemäßen Verfahrens (b) innerhalb eines größeren Bereichs variiert werden. Im allgemeinen arbeitet man bei Temperaturen zwischen 0°C und 150°C, vorzugsweise zwischen 10°C und 130°C.

Auch bei der Durchführung des erfindungsgemäßen Verfahrens (b) arbeitet man im allgemeinen unter Atmosphärendruck. Es ist aber auch möglich, unter erhöhtem oder vermindertem Druck zu arbeiten.

Bei der Durchführung des erfindungsgemäßen Verfahrens (b) setzt man auf 1 Mol an 3,4-Dichlor-isothiazol-5-carbonsäureamid der Formel (IV) im allgemeinen 1 bis 2 Mol, vorzugsweise 1 bis 1,5 Mol an Hydroxyverbindung der Formel (V) sowie 2 bis 6 Mol an wasserabspaltehdem Mittel ein. Die Aufarbeitung erfolgt nach üblichen Methoden. Im allgemeinen verfährt man in der Weise, daß man das Reaktionsgemisch mit Wasser versetzt, dann mit einem mit Wasser wenig mischbaren organischen Solvens extrahiert, die vereinigten organischen Phasen trocknet und unter vermindertem Druck einengt. Das verbleibende Produkt kann nach üblichen Methoden von eventuell noch vorhandenen Verunreinigungen befreit werden.

Die erfindungsgemäßen Wirkstoffe weisen eine starke pflanzenstärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, daß die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die erfindungsgemäßen Wirkstoffe weisen neben der pflanzenstärkenden (resistenzinduzierenden) Wirkung auch eine starke mikrobizide Wirkung auf und werden auch zur direkten Bekämpfung von unerwünschten Mikroorganismen praktisch eingesetzt. Die Wirkstoffe sind für den Gebrauch als Pflanzenschutzmittel, insbesondere als Fungizide geeignet.

Zu den unerwünschten Mikroorganismen im Pflanzenschutz gehören Pilze aus den Klassen Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, oder von Krankheiten im Wein-, Obst- und Gemüseanbau, wie beispielsweise gegen Plasmopara- oder Venturia-Arten, oder von Reiskrankheiten, wie beispielsweise gegen Pyricularia-Arten einsetzen. Mit gutem Erfolg lassen sich mit den erfindungsgemäßen Wirkstoffen auch weitere Pflanzenkrankheiten, wie beispielsweise Septoria-, Cochliobolus-, Pyrenophora- und Pseudocercosporella-Arten bekämpfen, wobei Drechslera teres speziell genannt sei.

Die erfindungsgemäßen Wirkstoffe eignen sich auch zur Steigerung des Ernteertrages. Sie sind außerdem mindertoxisch und weisen eine gute Pflanzenverträglichkeit auf.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sülfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0, und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Bendicar, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB), Quinoxyfen,
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon, (E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester,
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat, eis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N- (2,3 -Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin-Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on.

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaphorthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Elfusilanate, Emamectin, Empenthrin, Endosulfan, Entomopfthora spp., Esfenvalerate, Ethiofencarb, Ethion, Ethoprophos, Etofenprox, Etoxazole, Etrimphos,
Fenamiphos, Fenazaquin, Fenbutatinoxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren,
Halofenozide, HCH, Heptenophos, Hexaflumuron, Hexythiazox, Hydroprene,
Imidacloprid, Isazophos, Isofenphos, Isoxathion, Ivermectin,
Kernpolyederviren,
Lambda-cyhalothrin, Lufenuron,
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, No-valuron,
Omethoat, Oxamyl, Oxydemethon M,
Paeciloinyces fumosoroseus, Parathion A, Parathion M, Permethrin, Phenthoat, Phorat, Phosalon, Phosmet, Phosphamidon, Phoxim, Pirimicarb, Pirimiphos A, Pirimiphos M, Profenophos, Promecarb, Propoxur, Prothiophos, Prothoat, Pymetrozine, Pyraclofos, Pyresmethrin, Pyrethrum, Pyridaben, Pyridathion, Pyrimidifen, Pyriproxifen,
Quinalphos,
Ribavirin,
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanü
YI 5302
Zeta-Cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat,
(3-Phenoxyphenyl)-methyl-2,2,3,3-tetramethylcyclopropanecarboxylat, 1-[(2-Chlor-5-thiazolyl)methyl]tetrahydro-3,5-dimethyl-N-nitro-1,3,5-triazin-2(1H)-imin,
2-(2-Chlor-6-fluorphenyl)-4-[4-(1,1-diemthylethyl)phenyl]-4,5-dihydro-oxazol,
2-(Acetlyoxy)-3-docecyl-1,4-naphthalinidion,
2-Chlor-N-[[[4-(1-phenylethoxy)-phenyl]-amino]-carbonyl]-benzamid,
2-Chlor-N-[[[4-(2,2-dichlor-1,1-difluorethoxy)-phenyl]-amino]-carbonyl]-benzamid,
3-Methylphenyl-propylcarbamat,
4-[4-(4-Ethoxyphenyl)-4-methylpentyl]-1-fluor-2-phenoxy-benzol,
4-Chlor-2-(1,1-dimethylethyl)-5-[[2-(2,6-dimethyl-4-phenoxyphenoxy)ethyl]thio]-3(2H)-pyridazinon,
4-Chlor-2-(2-chlor-2-methylpropyl)-5-[(6-iod-3-pyridinyl)methoxy]-3(2H)-pyridazinon,
4-Chlor-5[(6-chlor-3-pyridinyl)methoxy]-2-(3,4-dichlorphenyl)-3(2H)-pyridazinon, Bacillus thuringiensis strain EG-2348,
Benzoesäure (2-benzoyl-1-(1,1-dimethylethyl)-hydrazid,
Butan 2,2-dimethyl-3-(2,4-dichlorphenyl)-2-oxo-1-oxaspiro[4,5]dec-3-en-4-yl-ester, [3-[(6-Chlor-3-pyridinyl)methyl]-2-thiazolidinyliden]-cyanamid,
Dihydro-2-(nitromethylen)-2H-1,3-thiazine-3(4H)-carboxaldehyd,
Ethyl-[2-[[1,6-dihydro-6-oxo-1-(phenylmethyl)-4-pyridazinyl]oxy]ethyl]-carbamat,
N-(3,4,4-Trifluor-1-oxo-3-butenyl)-glycin,
N-(4-Chlorphenyl)-3-[4-Difluormethoxy)phenyl]-4,5-dihydro-4-phenyl-1H-pyrazol-1-carboxamid,
N-[(2-Chlor-5-thiazolyl)methyl]-N'-methyl-N"-nitro-guanidin,
N-Methyl-N'-(1-methyl-2-propenyl)-1,2-hydrazindicarbothioamid,
N-Methyl-N'-2-propenyl-1,2-hydrazindicarbothioamid,
O,O-Diethyl-[2-(dipropylamino)-2-oxoethyl]-ethylphosphoramidothioat.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe zur Bekämpfung von Mikroorganismen können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Auch beim Einsatz gegen tierische Schädlinge können die erfindungsgemäßen Stoffe in handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die Herstellung und die Verwendung der erfindungsgemäßen Wirkstoffe wird durch die folgenden Beispiele veranschaulicht.

### Herstellungsbeispiele

### Beispiel 1

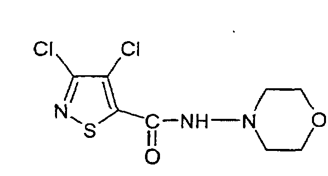

In ein Gemisch aus 1,21 g (0,0115 Mol) 4-Amino-morpholin und 22 ml absolutem Pyridin werden bei 0 bis 5°C unter Eiskühlung und unter Rühren 2,43 g (0,01 mol) 3,4-Dichlor-isothiazol-5-carbonsäurechlorid innerhalb von 5 Minuten eingetropft. Nach beendeter Zugabe versetzt man das Reaktionsgemisch mit 1,5 ml absolutem Tetrahydrofuran, läßt auf Raumtemperatur erwärmen und rührt dann eine Stunde bei Raumtemperatur. Anschließend wird das Reaktionsgemisch unter vermindertem Druck eingeengt. Der verbleibende Rückstand wird mit 60 ml Wasser und 60 ml Essigsäureethylester versetzt. Die organische Phase wird abgetrennt, und die wäßrige Phase wird noch zweimal mit Essigsäureethylester extrahiert. Die vereinigten organischen Phasen werden nacheinander mit 20 ml gesättigter, wäßriger Natriumhydrogencarbonat-Lösung und mit 50 ml Wasser gewaschen, dann über Natriumsulfat getrocknet und unter vermindertem Druck eingeengt. Der Rückstand wird in einem Gemisch aus 30 ml Methylenchlorid und 70 ml Petrolether verrieben. Das anfallende Festprodukt wird abgesaugt und getrocknet. Man erhält auf diese Weise 1,79 g (63 % der Theorie) an 3,4-Dichlor-isothiazol-5-carbonsäure-(morpholin-4-yl)-amid in Form einer Festsubstanz vom Schmelzpunkt 198 bis 199°C.

### Beispiel 2

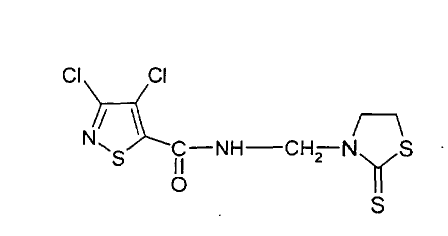

In ein Gemisch aus 3,0 g (0,015 Mol) 3,4-Dichlor-isothiazol-5-carbonsäureamid und 22,5 ml Eisessig werden unter Rühren bei Raumtemperatur 2,3 g (0,015 Mol) 3-Hydroxymethyl-thiazolidin-2-thion gegeben. Danach tropft man unter Rühren bei Raumtemperatur 3,27 g (0,033 Mol) konzentrierte Schwefelsäure hinzu, wobei das Reaktionsgemisch mit Eis gekühlt wird. Das Reaktionsgemisch wird zunächst 21 Stunden bei Raumtemperatur gerührt, dann mit 5 ml Eisessig versetzt und weitere 3 Stunden bei Raumtemperatur gerührt. Man versetzt unter Eiskühlung mit 25 ml Wasser und filtriert den anfallenden Feststoff ab. Der Filterrückstand wird mit 10 ml Wasser und 20 ml Petrolether gewaschen, dann in Methylenchlorid gelöst und mit Methylenchlorid als Laufmittel an Kieselgel chromatographiert. Nach dem Einengen des Eluates unter vermindertem Druck erhält 0,24 g (5 % der Theorie) an 3,4-Dichlor-isothiazol-5-carbonsäure-(thiazolidin-2-thion-3-yl-methyl)-amid in Form einer Festsubstanz vom Schmelzpunkt 181 bis 182°C.

Nach den zuvor angegebenen Methoden werden auch die in der folgenden Tabelle aufgeführten Isothiazol-carbonsäureamide der Formel (I) hergestellt.

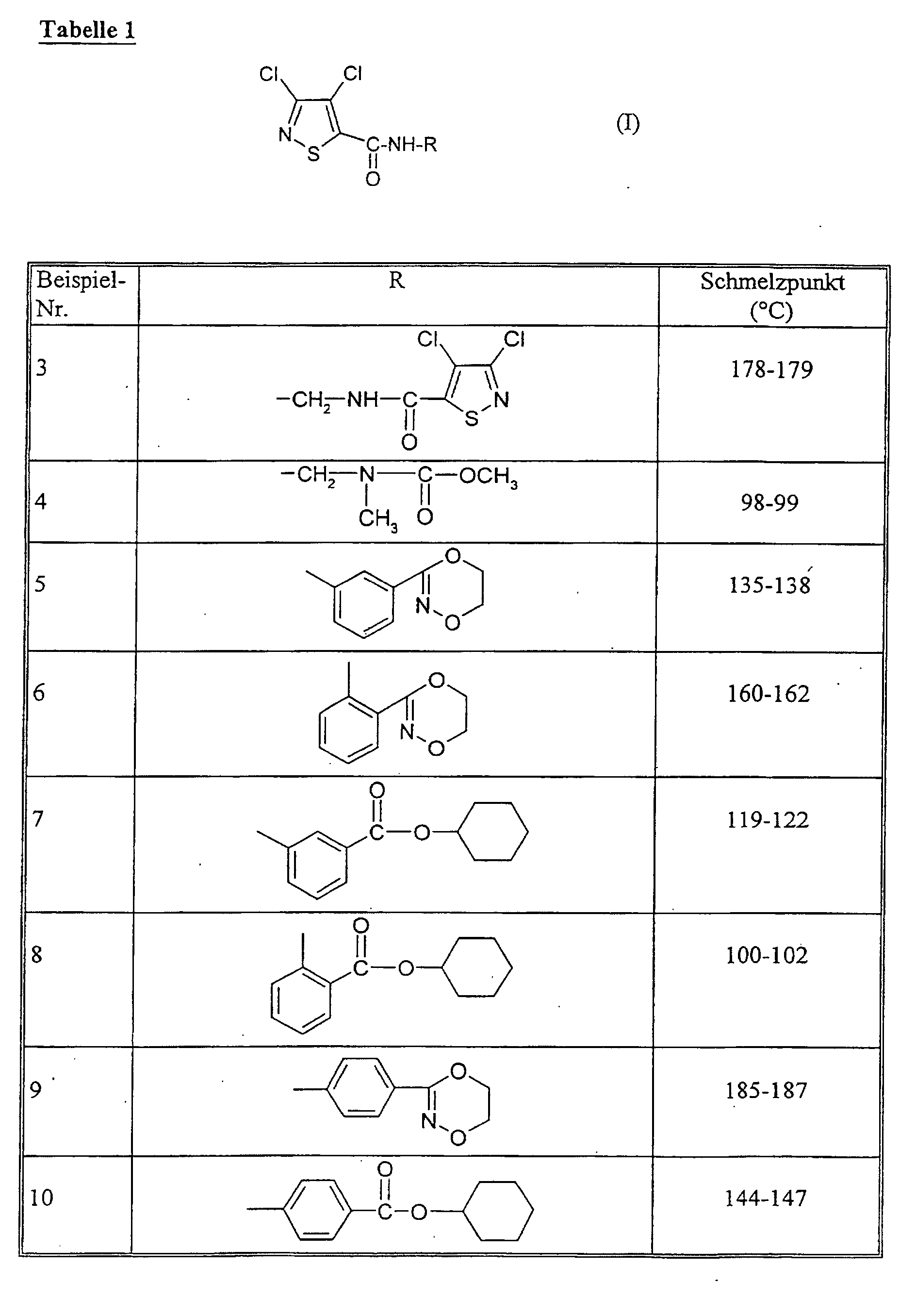

### Verwendungsbeispiel

### Beispiel A

### Erysiphe-Test (Gerste) / Resistenzinduktion

| | | | |
|---|---|---|---|
| Lösungsmittel | 50 | Gewichtsteile | N,N-Dimethylibrmamid |
| Emulgator | 1,17 | Gewichtsteile | Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf resistenzinduzierende Wirksamkeit bespritzt man junge Getreidepflanzen mit der Wirkstoffzubereitung in der angegebenen Aufwandmenge. 4 Tage nach der Behandlung werden die Pflanzen mit Sporen von Erysiphe graminis f. sp. hordei inokuliert. Anschließend werden die Pflanzen in einem Gewächshaus bei 70 % relativer Luftfeuchtigkeit und einer Temperatur von 18°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

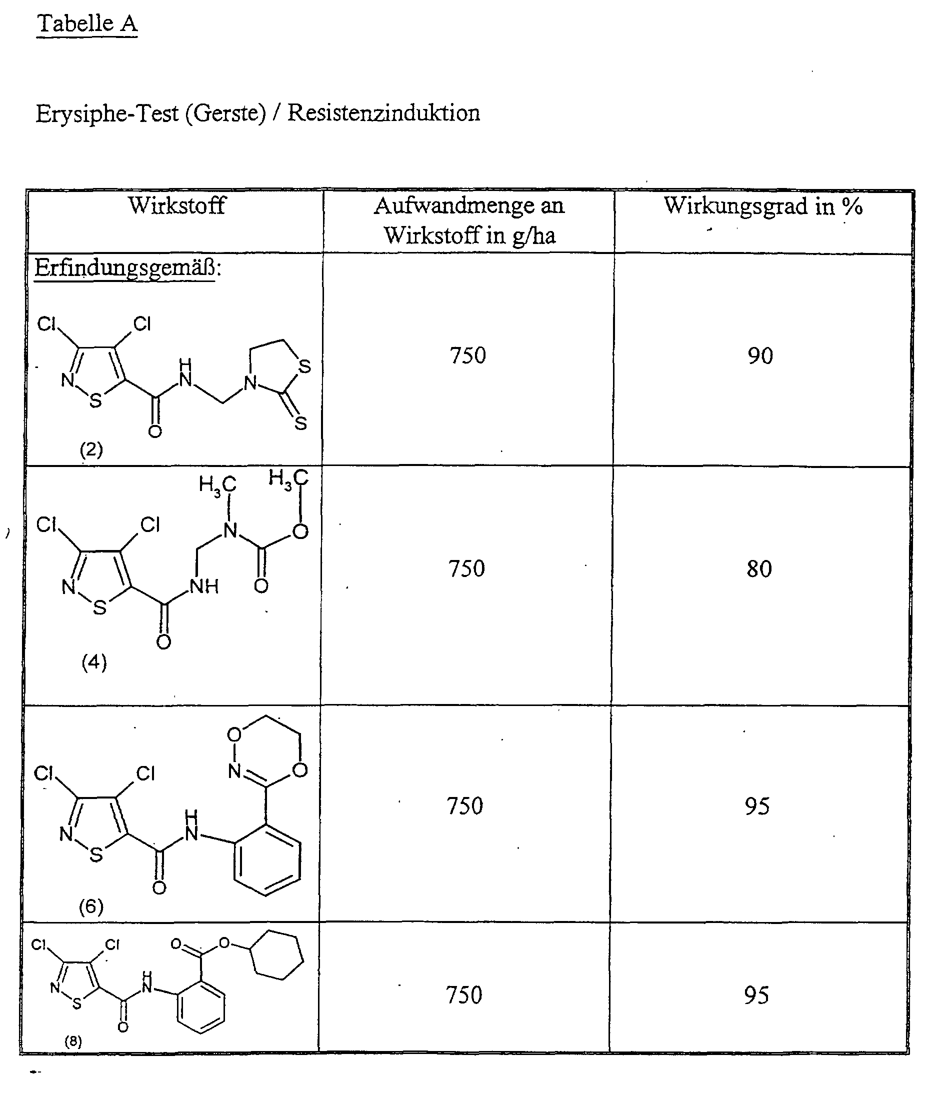

### Beispiel B

Erysiphe-Test (Gerste) / Resistenzinduktion

| | | | |
|---|---|---|---|
| Lösungsmittel | 97,6 | Gewichtsteile | N,N-Dimethylformamid |
| Emulgator | 2,4 | Gewichtsteile | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf resistenzinduzierende Wirksamkeit bespritzt man junge Getreidepflanzen mit der Wirkstoffzubereitung in der angegebenen Aufwandmenge. 4 Tage nach der Behandlung werden die Pflanzen mit Sporen von Erysiphe graminis f. sp. hordei inokuliert. Anschließend werden die Pflanzen in einem Gewächshaus bei 70 % relativer Luftfeuchtigkeit und einer Temperatur von 18°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

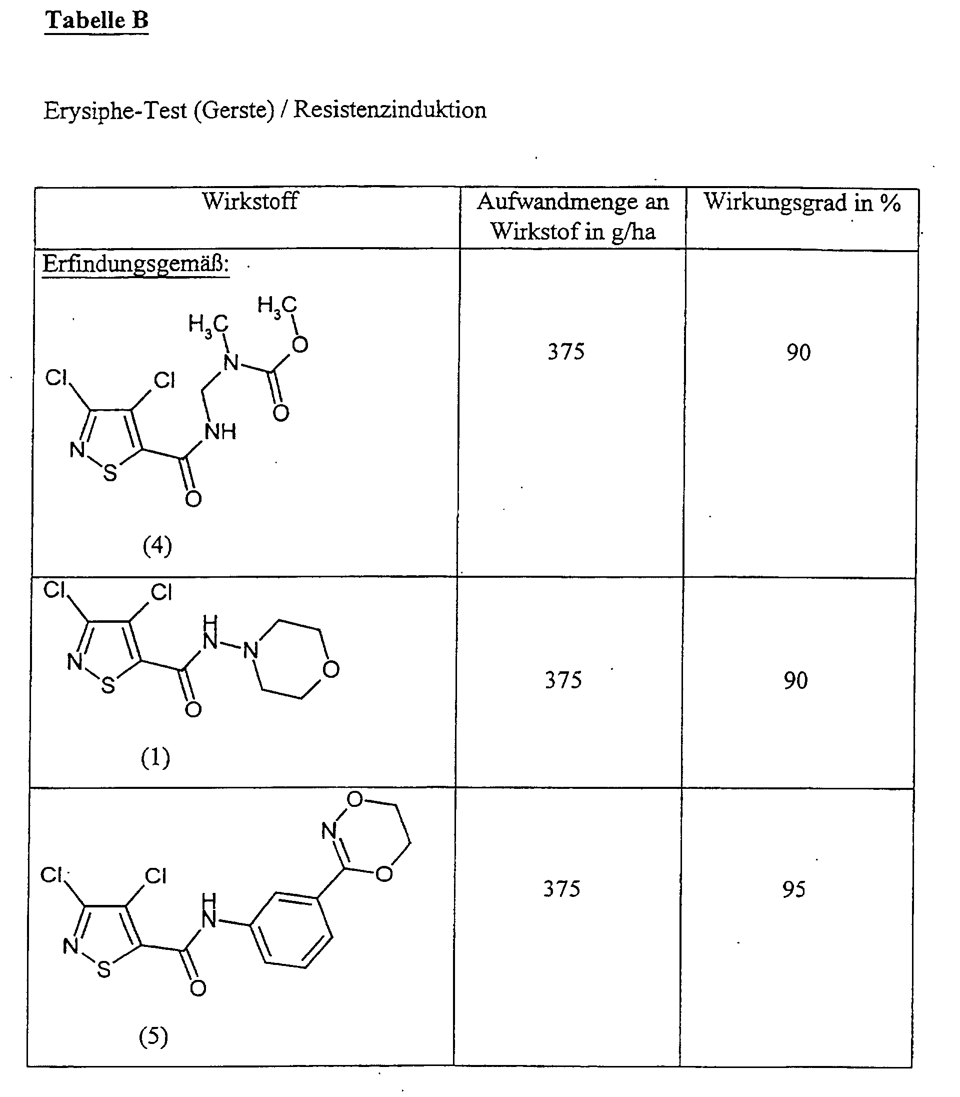

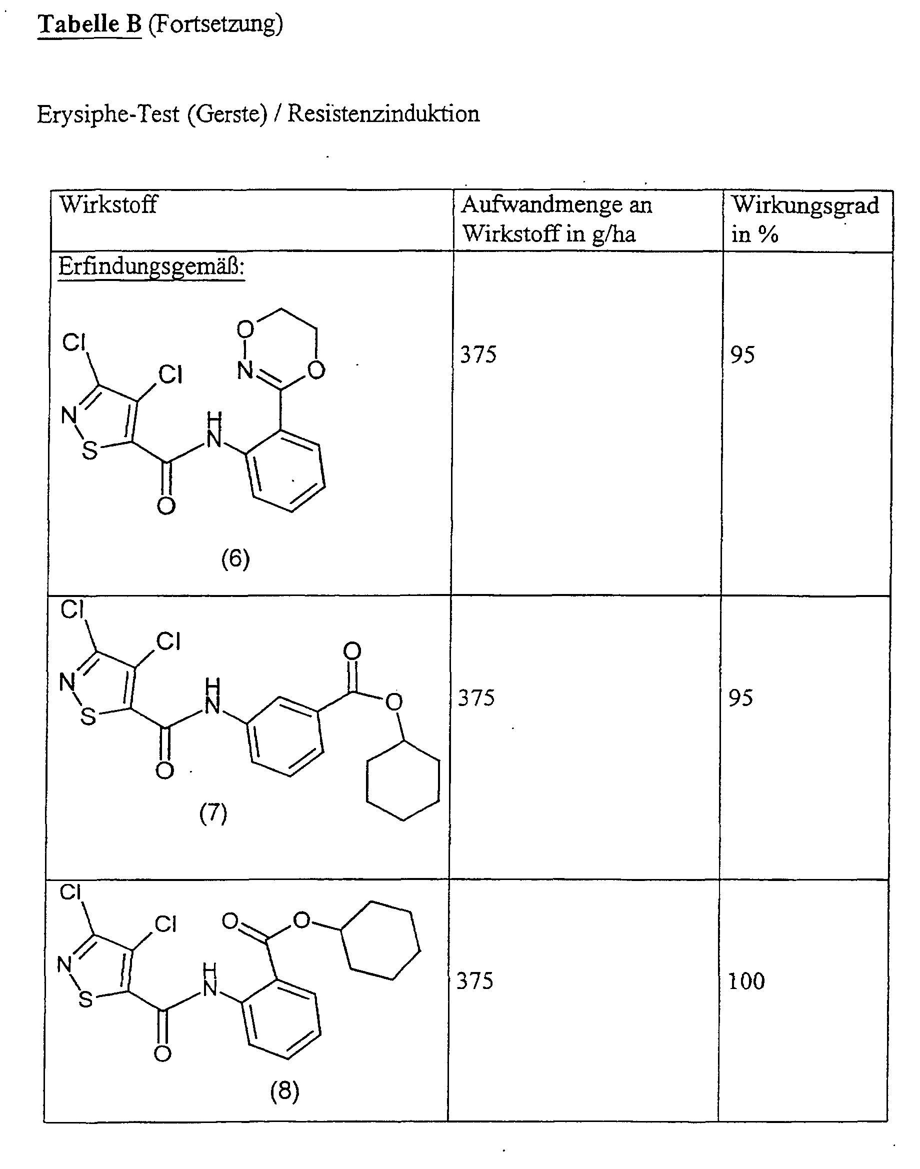

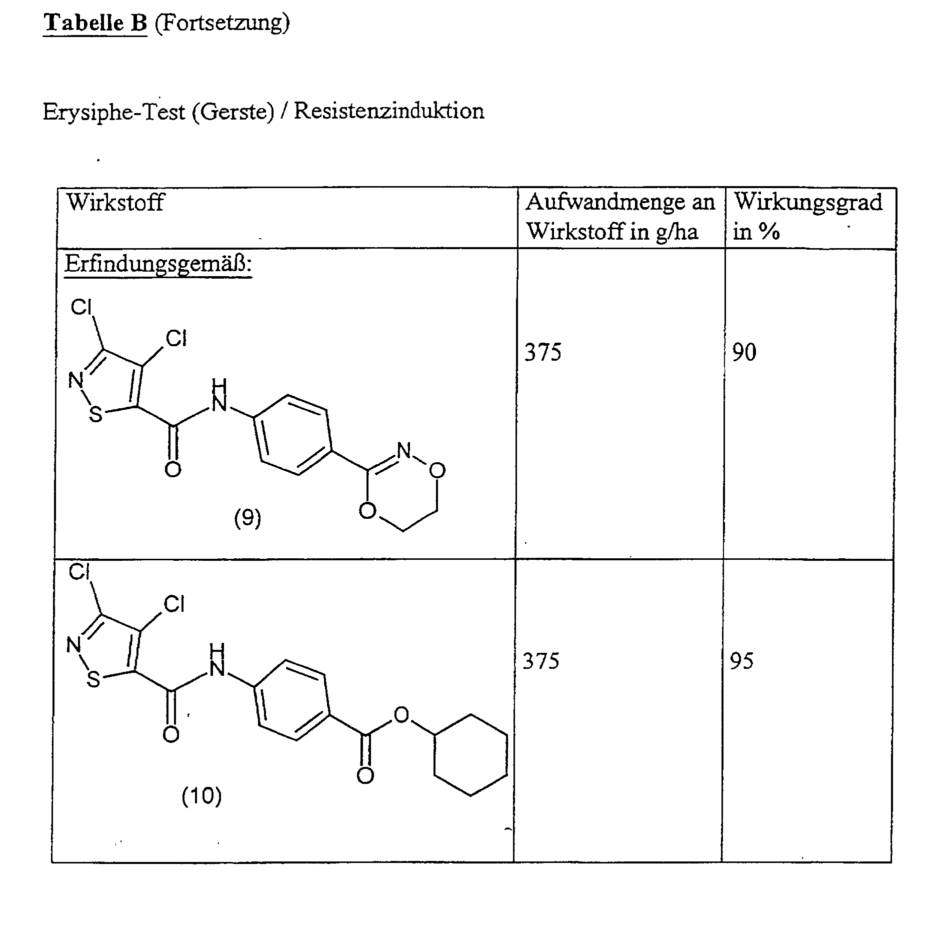

### Beispiel C

Pyricularia-Test (Reis) / protektiv

| | | | |
|---|---|---|---|
| Lösungsmittel | 97,6 | Gewichtsteile | N,N-Dimethylformamid |
| Emulgator | 2,4 | Gewichtsteile | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Reispflanzen mit der Wirkstoffzubereitung in der angegebenen Aufwandmenge. Nach dem Antrocknen des Spritzbelages werden die Pflanzen mit einer wäßrigen Sporensuspension von Pyricularia oryzae inokuliert und verbleiben dann 24 h bei 100 % rel. Luftfeuchte und 26°C. Anschließend werden die Pflanzen in einem Gewächshaus bei 80 % rel. Luftfeuchtigkeit und einer Temperatur von 26°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

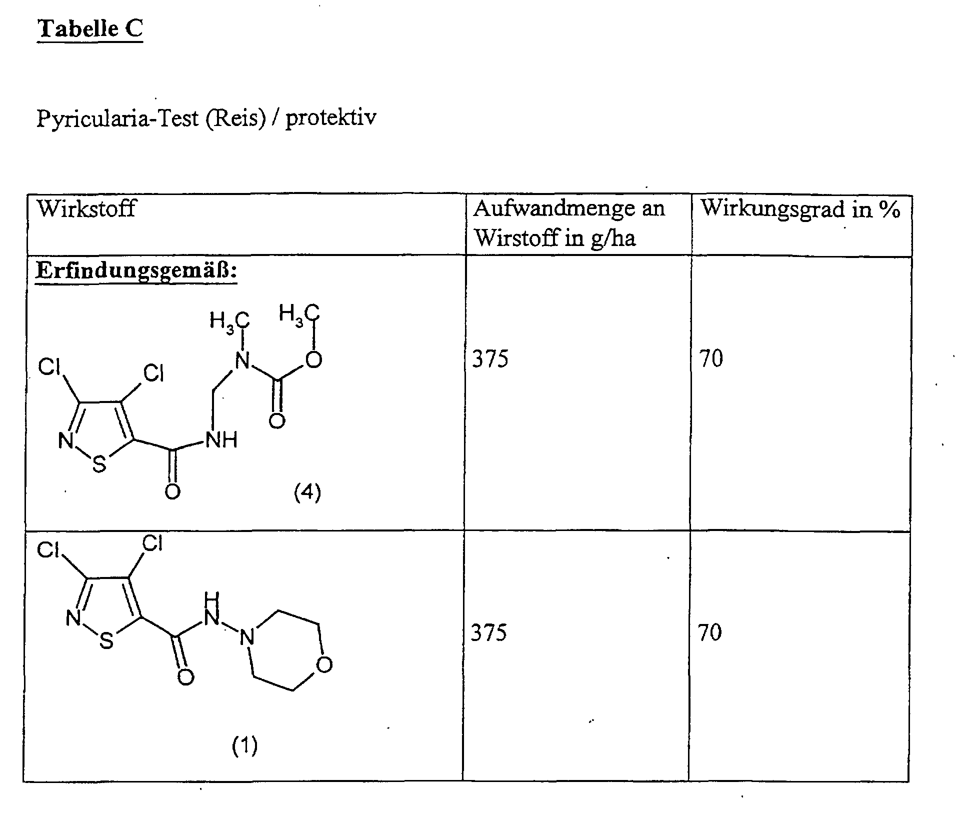

## Patentansprüche

1. Isothiazolcarbonsäureamide der Formel 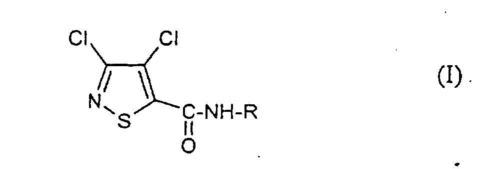 in welcher
R für einen Rest der Formel
-A¹-R⁵
steht, worin
A¹ für -CH₂-, -CH₂-CH₂- oder 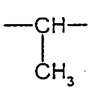 steht und
R⁵ für Thiazolidinthion-3-yl, Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl, 1,2,3-Triazolyl, Benzotriazolyl, Chinolinyl, Isochinolinyl, Chinazolinyl, Indolyl, Benzothienyl, Benzofuranyl, Benzothiazolyl oder Benzimidazolyl steht, wobei diese Reste einfach oder zweifach, gleichartig oder verschieden substituiert sein können durch Fluor, Chlor, Brom, Cyano, Nitro, Carboxyl, Methyl, Ethyl, Propyl, Isopropyl, n-Butyl, sek.-Butyl, iso-Butyl, tert.-Butyl, Halogenalkyl mit 1 oder 2 Kohlenstoffatomen und 1 bis 3 Fluor-, Chlor- und/oder Bromatomen, Methoxycarbonyl, Ethoxycarbonyl, Methoxy, Ethoxy, Propoxy, Isopropoxy, tert.-Butoxy, Halogenalkoxy mit 1 oder 2 Kohlenstoffatomen und 1 bis 3 Fluor-, Chlor- und/oder Bromatomen, Methylthio, Ethylthio, Halogenalkylthio mit 1 oder 2 Kohlenstoffatomen und 1 bis 3 Fluor-, Chlor- und/oder Bromatomen, Methylamino, Ethylamino, Dimethylamino, Diethylamino, Phenyl und/oder Phenoxy,
oder
R für einen Rest der Formel 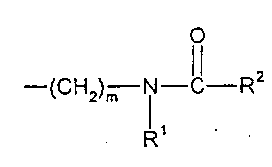 steht, worin
R¹ für Wasserstoff oder Alkyl mit 1 bis 4 Kohlenstoffatomen steht,
R² für Alkoxy mit 1 bis 4 Kohlenstoffatomen steht oder für Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl oder 1,2,3-Triazolyl steht, wobei diese Reste einfach oder zweifach, gleichartig oder verschieden substituiert sein können durch Fluor, Chlor, Brom, Cyano, Nitro, Carboxyl, Methyl; Ethyl, Propyl, Isopropyl, n-Butyl, sek.-Butyl, iso-Butyl, tert.-Butyl, Halogenalkyl mit 1 oder 2 Kohlenstoffatomen und 1 bis 3 Fluor-, Chlor- und/oder Bromatomen, Methoxycarbonyl, Ethoxycarbonyl, Methoxy, Ethoxy, Propoxy, Isopropoxy, tert.-Butoxy, Halogenalkoxy mit 1 oder 2 Kohlenstoffatomen und 1 bis 3 Fluor-, Chlor- und/oder Bromatomen, Methylthio, Ethylthio, Halogenalkylthio mit 1 oder 2 Kohlenstoffatomen und 1 bis 3 Fluor-, Chlor- und/oder Bromatomen, Methylamino, Ethylamino, Dimethylamino, Diethylamino, Phenyl und/oder Phenoxy,
und
m für 1 oder 2 steht,
oder
R für einen Rest der Formel 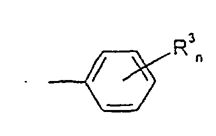 steht, worin
R³ für einen Heterocyclus der Formel 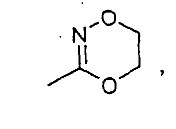 für Morpholinyl, Piperazinyl, Pyrazolyl, Imidazolyl, 1,2,4-Triazolyl, Pyrrolyl, Thienyl, Thiazolyl, Isothiazolyl, Oxazolyl, Tsoxazolyl, Pyridinyl, Pyrimidinyl, Triazinyl oder 1,2,3-Triazolyl swteht, wobei diese Reste einfach oder zweifach, gleichartig oder verschieden substituiert sein können durch Fluor, Chlor, Brom, Cyano, Nitro, Carboxyl, Methyl, Ethyl, Propyl, Isopropyl, n-Butyl, sek.-Butyl, iso-Butyl, tert.-Butyl, Halogenalkyl mit 1 oder 2 Kohlenstoffatomen und 1 bis 3 Fluor-, Chlor- und/oder Bromatomen, Methoxycarbonyl, Ethoxycarbonyl, Methoxy, Ethoxy, Propoxy, Isopropoxy, tert.-Butoxy, Halogenalkoxy mit 1 oder 2 Kohlenstoffatomen und 1 bis 3 Fluor-, Chlor- und/oder Bromatomen, Methylthio, Ethylthio, Halogenalkylthio mit 1 oder 2 Kohlenstoffatomen und 1 bis 3 Fluor-, Chlor- und/oder Bromatomen, Methylamino, Ethylamino, Dimethylamino, Diethylamino, Phenyl und/oder Phenoxy
oder
R³ für einen Rest der Formel 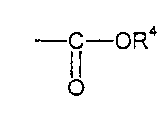 steht, worin
R⁴ für Cyclopropyl, Cyclopentyl oder Cyclohexyl steht
und
n für 1 oder 2 steht,
oder
R für Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl, 1,2,3-Triazolyl, Benzotriazolyl, Chinolinyl, Isochinolinyl, Chinazolinyl, Indolyl, Benzothienyl, Benzofuranyl, Benzothiazolyl oder Benzimidazolyl steht, wobei diese Reste einfach oder zweifach, gleichartig oder verschieden substituiert sein können durch Fluor, Chlor, Brom, Cyano, Nitro, Carboxyl, Methyl, Ethyl, Trifluormethyl, Difluormethyl, Trichlormethyl, Methoxycarbonyl, Ethoxycarbonyl, Methoxy, Ethoxy, Trifluormethoxy, Methylthio, Trifluormethylthio, Methylamino, Dimethylamino, Phenyl und/oder Phenoxy.

2. Verfahren zur Herstellung von Isothiazolcarbonsäureamiden der Formel (I) gemäß Anspruch 1, **dadurch gekennzeichnet, daß** man
a) 3,4-Dichlor-isothiazol-5-carbonsäurechlorid der Formel 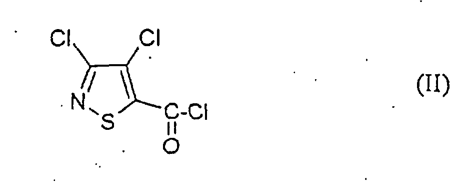 mit Aminen der Formel
H₂N-R (III)
in welcher
R die oben angegebenen Bedeutungen hat,
gegebenenfalls in Gegenwart eines Säurebindemittels und gegebenenfalls in Gegenwart eines Verdünnungsmittels umsetzt,
oder
b) 3,4-Dichlor-isothiazol-5-carbonsäureamid der Formel 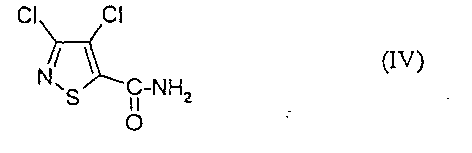 mit Hydroxyverbindungen der Formel
HO-A-X (V)
in welcher
X für Thiazolidinthion-3-yl, Morpholinyl, Piperazinyl, Pyrazolyl, Imidazolyl, 1,2,4-Triazolyl, Pyrrolyl, Thienyl, Thiazolyl, Isothiazolyl, Oxazolyl, Isoxazolyl, Pyridinyl, Pyrimidinyl, Triazinyl, 1,2,3-Triazolyl, Benzotriazolyl, Chinolinyl, Isochinolinyl, Chinazolinyl, Indolyl, Benzothienyl, Benzofuranyl, Benzothiazolyl oder Benzimidazolyl steht, wobei diese Reste einfach oder zweifach, gleichartig oder verschieden substituiert sein können durch Fluor, Chlor, Brom, Cyano, Nitro, Carboxyl, Methyl, Ethyl, Propyl, Isopropyl, n-Butyl, sek.-Butyl, iso-Butyl, tert.-Butyl, Halogenalkyl Halogenalkyl mit 1 bis 2 Kohlenstoffatomen und I bis 3 Fluor-, Chlor- und/oder Bromatomen, Methoxycarbonyl, Ethoxycarbonyl, Methoxy, Ethoxy, Propoxy, Isopropoxy, tert.-Butoxy, Halogenalkoxy mit 1 bis 2 Kohlenstoffatomen und 1 bis 3 Fluor-, Chlor- und/oder Bromatomen, Methylthio, Ethylthio, Halogenalkylthio mit 1 bis 2 Kohlenstoffatomen und 1 bis 3 Fluor-, Chlor- und/oder Bromatomen, Methylamino, Ethylamino, Dimethylamino, Diethylamino, Phenyl und/oder Phenoxy,
oder
X für einen Rest der Formel 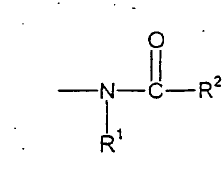 steht, worin
R¹ und R² diejenigen Bedeutungen haben, die im Zusammenhang mit der Beschreibung der erfindungsgemäßen Stoffe der Formel (I) für diese Reste genannt wurden;
und
A für CH₂-, -CH₂-CH₂- oder 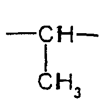 steht,
in Gegenwart eines Verdünnungsmittels und in Gegenwart eines wasser-abspaltenden Mittels umsetzt.

3. Mittel zum Schutz von Pflanzen gegen Befall durch unerwünschte Mikroorganismen, **gekennzeichnet durch** einen Gehalt an mindestens einem [sothiazolcarbonsäureamid der Formel (I) gemäß Anspruch 1 neben Streckmitteln und/oder oberflächenaktiven Stoffen.

4. Verwendung von Isothiazolcarbonsäureamiden der Formel (I) gemäß Anspruch 1 zum Schutz von Pflanzen gegen Befall durch unerwünschte Mikroorganismen.

5. Verfahren zum Schutz von Pflanzen gegen Befall durch unerwünschte Mikroorganismen, **dadurch gekennzeichnet, daß** man Isothiazolcarbonsäureamide der Formel (I) gemäß Anspruch 1 auf die Pflanzen und/oder deren Lebensraum ausbringt.

6. Verfahren zur Herstellung von Mitteln zum Schutz von Pflanzen gegen Befall durch unerwünschte Mikroorganismen, **dadurch gekennzeichnet, daß** man Isothiazolcarbonsäureamide der Formel (I) gemäß Anspruch 1 mit Streckmitteln und/oder oberflächenaktiven Stoffen vermischt.

7. Isothiazolcarbonsäureamid gemäß Anspruch 1, **gekennzeichnet durch** die Formel

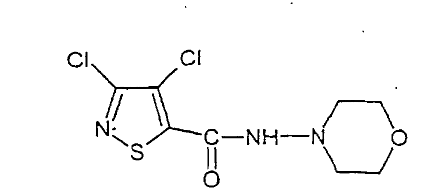

8. Isothiazolcarbonsäureamid gemäß Anspruch 1, **gekennzeichnet durch** die Formel

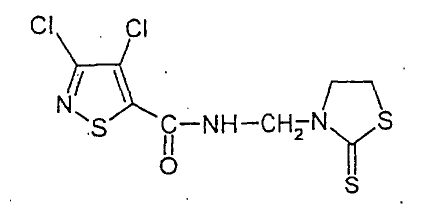

9. Isothiazolcarbonsäureamid gemäß Anspruch 1, **gekennzeichnet durch** die Formel

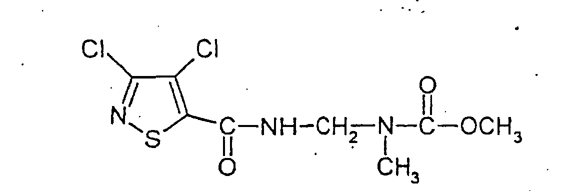

10. Isothiazolcarbonsäureamid gemäß Anspruch 1, **gekennzeichnet durch** die Formel

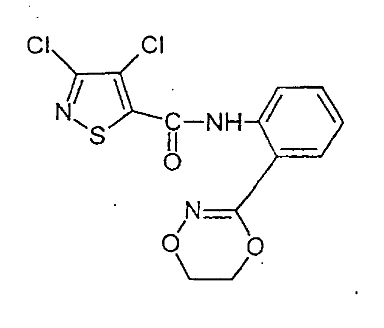

## Claims

1. Isothiazolecarboxamide of the formula 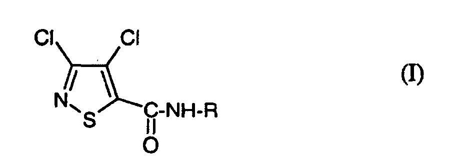 in which
R represents a radical of the formula
-A¹-R⁵,
in which
A¹ represents -CH₂-, -CH₂-CH₂- or 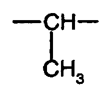 and
R⁵ represents thiazolidinethion-3-yl, morpholinyl, piperazinyl, pyrazolyl, imidazolyl, 1,2,4-triazolyl, pyrrolyl, thienyl, thiazolyl, isothiazolyl, oxazolyl, isoxazolyl, pyridinyl, pyrimidinyl, triazinyl, 1,2,3-triazolyl, benzotriazolyl, quinolinyl, isoquinolinyl, quinazolinyl, indolyl, benzothienyl, benzofuranyl, benzothiazolyl or benzimidazolyl, where these radicals may be mono- or disubstituted by identical or different substituents from the group consisting of fluorine, chlorine, bromine, cyano, nitro, carboxyl, methyl, ethyl, propyl, isopropyl, n-butyl, sec-butyl, iso-butyl, tert-butyl, halogenoalkyl having 1 or 2 carbon atoms and 1 to 3 fluorine, chlorine and/or bromine atoms, methoxycarbonyl, ethoxycarbonyl, methoxy, ethoxy, propoxy, isopropoxy, tert-butoxy, halogenoalkoxy having I or 2 carbon atoms and I to 3 fluorine, chlorine and/or bromine atoms, methylthio, ethylthio, halogenoalkylthio having 1 or 2 carbon atoms and 1 to 3 fluorine, chlorine and/or bromine atoms, methylamino, ethylamino, dimethyl amino, diethylamino, phenyl and phenoxy,
or
R represents a radical of the formula 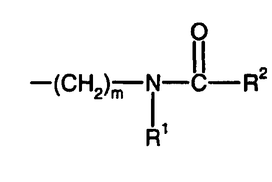 , in which
R¹ represents hydrogen or alkyl having 1 to 4 carbon atoms,
R² represents alkoxy having 1 to 4 carbon atoms or represents morpholinyl, piperazinyl, pyrazolyl, imidazolyl, 1,2,4-triazolyl, pyrrolyl, thienyl, thiazolyl, isothiazolyl, oxazolyl, isoxazolyl, pyridinyl, pyrimidinyl, triazinyl or 1,2,3-triazolyl, where these radicals may be mono- or disubstituted by identical or different substituents from the group consisting of fluorine, chlorine, bromine, cyano, nitro, carboxyl, methyl, ethyl, propyl, isopropyl, n-butyl, see-butyl, iso-butyl, tert-butyl, halogenoalkyl having 1 or 2 carbon atoms and 1 to 3 fluorine, chlorine and/or bromine atoms, methoxycarbonyl, ethoxycarbonyl, methoxy, ethoxy, propoxy, isopropoxy, tert-butoxy, halogenoalkoxy having 1 or 2 carbon atoms and 1 to 3 fluorine, chlorine and/or bromine atoms, methylthio, ethylthio, halogenoalkylthio having 1 or 2 carbon atoms and 1 to 3 fluorine, chlorine and/or bromine atoms, methylamino, ethylamino, dimethylamino, diethylamino, phenyl and phenoxy,
and
m represents 1 or 2,
or
R represents a radical of the formula 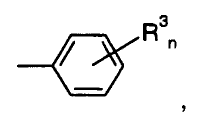 in which
R³ represents a heterocycle of the formula 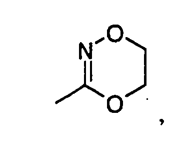 represents morpholinyl, piperazinyl, pyrazolyl, imidazolyl, 1,2,4-triazolyl, pyrrolyl, thienyl, thiazolyl, isothiazolyl, oxazolyl, isoxazolyl, pyridinyl, pyrimidinyl, triazinyl or 1,2,3-triazolyl, where these radicals may be mono- or disubstituted by identical or different substituents from the group consisting of fluorine, chlorine, bromine, cyano, nitro, carboxyl, methyl, ethyl, propyl, isopropyl, n-butyl, see-butyl, iso-butyl, tert-butyl, halogenoalkyl having 1 or 2 carbon atoms and 1 to 3 fluorine, chlorine and/or bromine atoms, methoxycarbonyl, ethoxycarbonyl, methoxy, ethoxy, propoxy, isopropoxy, tert-butoxy, halogenoalkoxy having 1 or 2 carbon atoms and 1 to 3 fluorine, chlorine and/or bromine atoms, methylthio, ethylthio, halogenoalkylthio having 1 or 2 carbon atoms and 1 to 3 fluorine, chlorine and/or bromine atoms, methylamino, ethylamino, dimethylamino, diethylamino, phenyl and phenoxy
or
R³ represents a radical of the formula 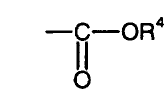 , in which
R⁴ represents cyclopropyl, cyclopentyl or cyclohexyl,
and
n represents 1 or 2,
or
R represents morpholinyl, piperazinyl, pyrazolyl, imidazolyl, 1,2,4-triazolyl, pyrrolyl, thienyl, thiazolyl, isothiazolyl, oxazolyl, isoxazolyl, pyridinyl, pyrimidinyl, triazinyl, 1,2,3-triazolyl, benzotriazolyl, quinolinyl, isoquinolinyl, quinazolinyl, indolyl, benzothienyl, benzofuranyl, benzothiazolyl or benzimidazolyl, where these radicals may be mono- or disubstituted by identical or different substituents from the group consisting of fluorine, chlorine, bromine, cyano, nitro, carboxyl, methyl, ethyl, trifluoromethyl, difluoromethyl, trichloromethyl, methoxycarbonyl, ethoxycarbonyl, methoxy, ethoxy, trifluoromethoxy, methylthio, trifluoromethylthio, methylamino, dimethylamino, phenyl and phenoxy.

2. Process for preparing isothiazolecarboxamides of the formula (I) according to Claim 1, **characterized in that**
a) 3,4-dichloro-isothiazole-5-carbonyl chloride of the formula 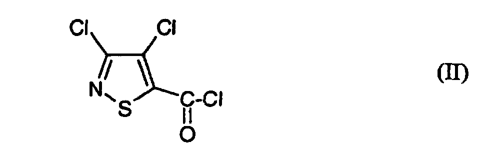 is reacted with amines of the formula
H₂N-R (III)
in which
R is as defined above,
if appropriate in the presence of an acid binder and if appropriate in the presence of a diluent,
or
b) 3,4-dichloro-isothiazole-5-carboxamide of the formula 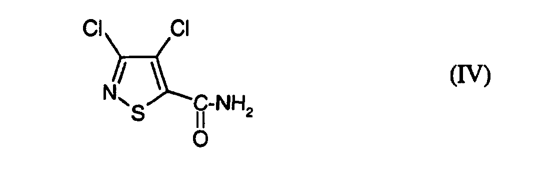 is reacted with hydroxyl compounds of the formula
HO-A-X (V)
in which
X represents thiazolidinethion-3-yl, morpholinyl, piperazinyl, pyrazolyl, imidazolyl, 1,2,4-triazolyl, pyrrolyl, thienyl, thiazolyl, isothiazolyl, oxazolyl, isoxazolyl, pyridinyl, pyrimidinyl, triazinyl, 1,2,3-triazolyl, benzotriazolyl, quinolinyl, isoquinolinyl, quinazolinyl, indolyl, benzothienyl, benzofuranyl, benzothiazolyl or benzimidazolyl, where these radicals may be mono- or disubstituted by identical or different substituents from the group consisting of fluorine, chlorine, bromine, cyano, nitro, carboxyl, methyl, ethyl, propyl, isopropyl, n-butyl, see-butyl, iso-butyl, tert-butyl, halogenoalkyl having 1 or 2 carbon atoms and 1 to 3 fluorine, chlorine and/or bromine atoms, methoxycarbonyl, ethoxycarbonyl, methoxy, ethoxy, propoxy, isopropoxy, tert-butoxy, halogenoalkoxy having 1 or 2 carbon atoms and 1 to 3 fluorine, chlorine and/or bromine atoms, methylthio, ethylthio, halogenoalkylthio having 1 or 2 carbon atoms and 1 to 3 fluorine, chlorine and/or bromine atoms, methylamino, ethylamino, dimethylamino, diethylamino, phenyl and phenoxy,
or
X represents a radical of the formula 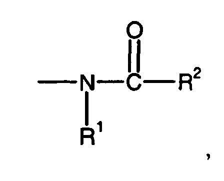 in which
R¹ and R² have those meanings which have been mentioned in connection with the description of the substances of the formula (I) according to the invention for these radicals,
and
A represents CH₂-, -CH₂-CH₂- or 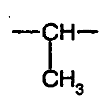 ,
in the presence of a diluent and in the presence of a dehydrating agent.

3. Composition for protecting plants against attack by undesirable microorganisms, **characterized in that** it contains at least one isothiazolecarboxamide of the formula (I) according to Claim 1, in addition to extenders and/or surfactants.

4. Use of isothiazolecarboxamides of the formula (I) according to Claim 1 for protecting plants against attack by undesirable micoorganisms.

5. Method for protecting plants against attack by undesirable microorganisms, **characterized in that** isothiazolecarboxamides of the formula (I) according to Claim 1 are applied to the plants and/or their habitat.

6. Process for preparing compositions for protecting plants against attack by undesirable microorganisms, **characterized in that** isothiazolecarboxamides of the formula (I) according to Claim 1 are mixed with extenders and/or surfactants.

7. Isothiazolecarboxamide according to Claim 1, **characterized by** the formula

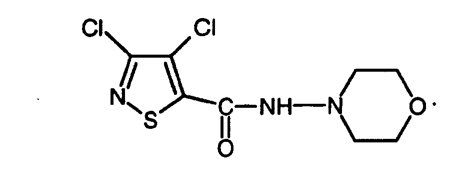

8. Isothiazolecarboxamide according to Claim 1, **characterized by** the formula

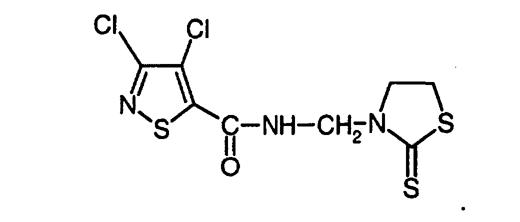

9. Isothiazolecarboxamide according to Claim 1, **characterized by** the formula

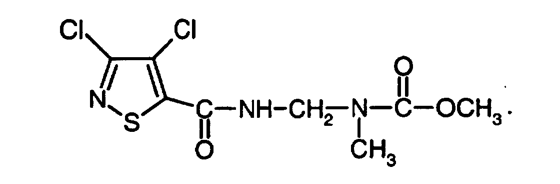

10. Isothiazolecarboxamide according to Claim 1, **characterized by** the formula

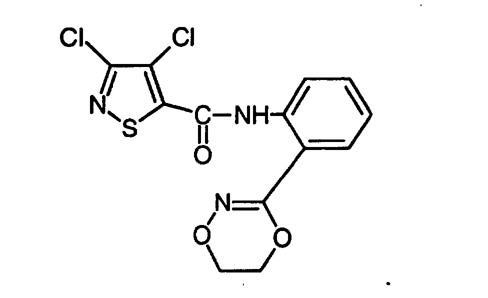

## Revendications

1. Isothiazolcarboxamides de formule 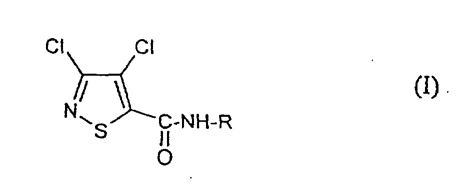 dans laquelle
R est un reste de formule
-A¹-R⁵,
dans laquelle
A¹ est un reste -CH₂-, -CH₂-CH₂-ou 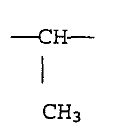
R⁵ est un reste thiazolidinethione-3-yle, morpholinyle, pipérazinyle, pyrazolyle, imidazolyle, 1,2,4-triazolyle, pyrrolyle, thiényle, thiazolyle, isothiazolyle, oxazolyle, isoxazolyle, pyridinyle, pyrimidinyle, triazinyle, 1,2,3-triazolyle, benzotriazolyle, quinolinyle, isoquinolinyle, quinazolinyle, indolyle, benzothiényle, benzofurannyle, benzothiazolyle ou benzimidazolyle, ces restes pouvant porter un ou deux substituants, identiques ou différents, fluore, chlore, brome, cyano, nitro, carboxyle, méthyle, éthyle, propyle, isopropyle, n-butyle, sec-butyle, isobutyle, tertiobutyle, halogénalkyle ayant un ou deux atomes de carbone et un à trois atomes de fluor, de chlore et/ou de brome, méthoxycarbonyle, éthoxycarbonyle, méthoxy, éthoxy, propoxy, isopropoxy, tertiobutoxy, halogénalkyle ayant un ou deux atomes de carbone et un à trois atomes de fluor, de chlore et/ou de brome, méthylthio, éthylthio, halogénalkylthio ayant un ou deux atomes de carbone et un à trois atomes de fluor, de chlore et/ou de brome, méthylamino, éthylamino, diméthylamino, diéthylamino, phényle et/ou phénoxy,
ou bien
R est un reste de formule 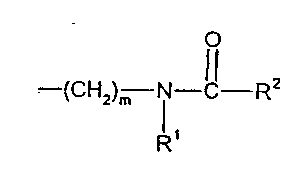 dans laquelle
R¹ est l'hydrogène ou un reste alkyle ayant un à quatre atomes de carbone,
R2 est un reste alkoxy ayant un à quatre atomes de carbone ou un reste morpholinyle, pipérazinyle, pyrazolyle, imidazolyle, 1,2,4-triazolyle, pyrrolyle, thiényle, thiazolyle, isothiazolyle, oxazolyle, isoxazolyle, pyridinyle, pyrimidinyle, triazinyle ou 1,2,3-triazolyle, ces restes pouvant porter un ou deux substituants, identiques ou différents, fluore, chlore, brome, cyano, nitro, carboxyle, méthyle, éthyle, propyle, isopropyle, n-butyle, sec-butyle, isobutyle, tertiobutyle, halogénalkyle ayant un ou deux atomes de carbone et un à trois atomes de fluor, de chlore et/ou dé brome, méthoxycarbonyle, éthoxycarbonyle, méthoxy, éthoxy, propoxy, isopropoxy, tertiobutoxy, halogénalkoxy ayant un ou deux atomes de carbone et un à trois atomes de fluor, de chlore et/ou de brome, méthylthio, éthylthio, halogénalkylthio m ayant un ou deux atomes de carbone et un à trois atomes de fluor, de chlore et/ou de brome, méthylamino, éthylamino, diméthylamino, diéthylamino, phényle et/ou phénoxy,
et
m a la valeur 1 ou 2,
ou bien
R représente un reste de formule 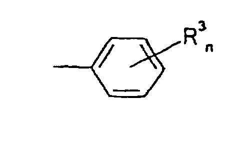 dans laquelle
R³ est un hétérocycle de formule 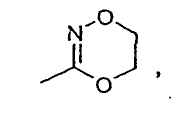 un reste morpholinyle, pipérazinyle, pyrazolyle, imidazolyle, 1,2,4-triazolyle, pyrrolyle, thiényle, thiazolyle, isothiazolyle, oxazolyle, isoxazolyle, pyridinyle, pyrimidinyle, triazinyle ou 1,2,3-triazolyle, ces restes pouvant porter un ou deux substituants, identiques ou différents, fluor, chlore, brome, cyano, nitro, carboxyle, méthyle, éthyle, propyle, isopropyle, n-butyle, sec-butyle, isobutyle, tertiobutyle, halogénalkyle ayant un ou deux atomes de carbone et un à trois atomes de fluor, de chlore et/ou de brome, méthoxycarbonyle, éthoxycarbonyle, méthoxy, éthoxy, propoxy, isopropoxy, tertiobutoxy, halogénalkoxy ayant un ou deux atomes de carbone et un à trois atomes de fluor, de chlore et/ou de brome, méthylthio, éthylthio, halogénalkylthio ayant un ou deux atomes de carbone et un à trois atomes de fluor, de chlore et/ou de brome, méthylamino, éthylamino, diméthylamino, diéthylamino, phényle et/ou phénoxy,
ou bien
R³ est un reste de formule 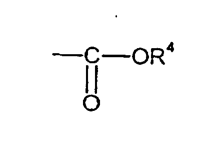 dans laquelle
R⁴ est un reste cyclopropyle, cyclopentyle ou cyclohexyle
et
n a la valeur 1 ou 2,
ou bien
R est un reste morpholinyle, pipérazinyle, pyrazolle, imidazolyle, 1,2,4-triazolyle, pyrrolyle, thiényle, thiazolyle, isothiazolyle, oxazolyle, isoxazolyle, pyridinyle, pyrimidinyle, triazinyle ou 1,2,3-triazolyle, benzotriazolyle, quinolinyle, isoquinolinyle, quinazolinyle, indolyle, benzothiényle, benzofurannyle, benzothiazolyle ou benzimidazolyle, ces restes pouvant porter un ou deux substituants, identiques ou différents, fluor, chlore, brome, cyano, nitro, carboxyle, méthyle, éthyle, trifluorométhyle, difluorométhyle, trichlorométhyle, méthoxycarbonyle, éthoxycarbonyle, méthoxy, éthoxy, trifluorométhoxy, méthylthio, trifluorométhylthio, méthylamino, diméthylamino, phényle et/ou phénoxy.

2. Procédé de production d'isothiazolcarboxamides de formule (I) suivant la revendication 1, **caractérisé en ce que** :
a) on fait réagir le chlorure d'acide 3,4-dichlorisothiazole-5-carboxylique de formule 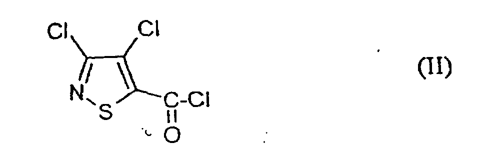 avec des amines de formule
H₂N-R
dans laquelle
R a la définition indiquée ci-dessus,
en présence éventuelle d'un accepteur d'acide et le cas échéant, en présence d'un diluant,
ou bien
b) on fait réagir le 3,4-dichloro-isothiazole-5-carboxamide de formule 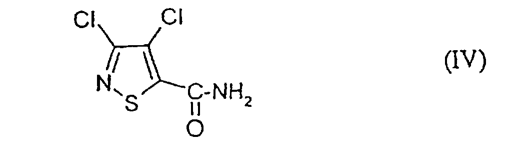 avec des composés hydroxylés de formule
HO-A-X
dans laquelle
X est un reste thiazolidinethione-3-yle, morpholinyle, pipérazinyle, pyrazolyle, imidazolyle, 1,2,4-triazolyle, pyrrolyle, thiényle, thiazolyle, isothiazolyle, oxazolyle, isoxazolyle, pyridinyle, pyrimidinyle, triazinyle, 1,2,3-triazolyle, benzotriazolyle, quinolinyle, isoquinolinyle, quinazolinyle, indolyle, benzothiényle, benzofurannyle, benzothiazolyle ou benzimidazolyle, ces restes pouvant porter un ou deux substituants, identiques ou différents, fluoré, chlore, brome, cyano, nitro, carboxyle, méthyle, éthyle, propyle, isopropyle, n-butyle, sec-butyle, isobutyle, tertiobutyle, halogénalkyle, halogénalkyle ayant un ou deux atomes de carbone et un à trois atomes de fluor, de chlore et/ou de brome, méthoxycarbonyle, éthoxycarbonyle, méthoxy, éthoxy, propoxy, isopropoxy, tertiobutoxy, halogénalkoxy ayant un ou deux atomes de carbone et un à trois atomes de fluor, de chlore et/ou de brome, méthylthio, éthylthio, halogénalkylthio ayant un ou deux atomes de carbone et un à trois atomes de fluor, de chlore et/ou de brome, méthylamino, éthylamino, diméthylamino, diéthylamino, phényle et/ou phénoxy,
ou bien
X représente un reste de formule 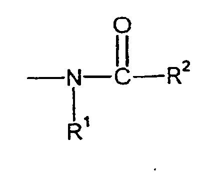
dans laquelle
R¹ et R² ont les définitions qui ont été mentionnées pour ces restes à propos de la description des composés de formule (I) conformes à l'invention
et
A est un reste -CH₂-, -CH₂-CH₂-ou 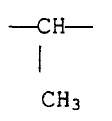 en présence d'un diluant et en présence d'un agent déshydratant.

3. Composition destinée à protéger les plantes de l'attaque par des micro-organismes indésirables, **caractérisée par** une teneur en au moins un isothiazolcarboxamide de formule (I) suivant la revendication 1, à côté de diluants et/ou d'agents tensioactifs.

4. Utilisation d'isothiazolcarboxamides de formule (I) suivant la revendication 1, pour protéger les plantes d'une attaque par des micro-organismes indésirables.

5. Procédé pour protéger des plantes d'une attaque par des micro-organismes indésirables, **caractérisé en ce qu'**on épand des isothiazolcarboxamides de formule (I) suivant la revendication 1 sur les plantes et/ou sur leur milieu.

6. Procédé de production de compositions destinées à protéger des plantes d'une attaque par des micro-organismes indésirables, **caractérisé en ce qu'**on mélange des isothiazolcarboxamides de formule (I) suivant la revendication 1 avec des diluants et/ou des agents tensioactifs.

7. Isothiazolcarboxamide suivant la revendication 1, **caractérisé par** la formule

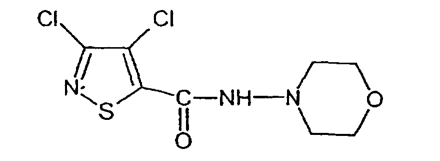

8. Isothiazolcarboxamide suivant la revendication 1, **caractérisé par** la formule

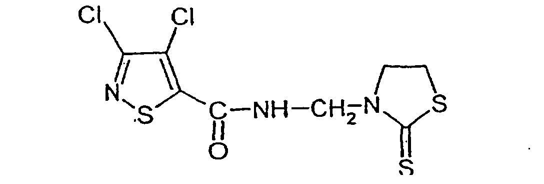

9. Isothiazolcarboxamide suivant la revendication 1, **caractérisé par** la formule

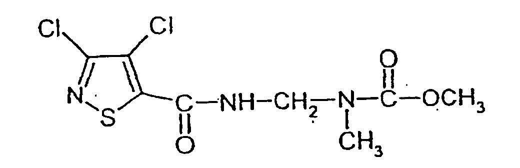

10. Isothiazolcarboxamide suivant la revendication 1, **caractérisé par** la formule
